# EUROPEAN PATENT APPLICATION

(11) **EP 2 848 273 A1**
(43) Date of publication of application: **18.03.2015**
(21) Application number: 13876947.6
(22) Date of filing: 04.03.2013
(51) Int. Cl.: A61M 5/315

(54) **PISTON MEMBER FOR SYRINGE**

(71) Applicant: Coki Engineering Inc., Osaka-shi, Osaka 540-0037 (JP)
(72) Inventor: YOTSUTSUJI, Akira, Osaka-shi Osaka 540-0037 (JP)
(74) Representative: Horn Kleimann Waitzhofer
(86) International application number: PCT/JP2013/001314
(87) International publication number: WO 2014/136138

(57) **Abstract**

Objective: To provide a low-cost piston member for syringes, with sufficiently satisfying slidability and sealing performance such as non-leakage and vapor impermeability, without size limitation, from small to large diameters.

Solution: A piston member 10, such as a gasket 10a or a middle piston 10b, is formed by cut-processing a PTFE block and is press-fitted in a syringe barrel 1 to be slidably used. Protruded rims 13 are formed at least in a circumferential direction of perimeter of a slide-contact surface 11a adjacent to a liquid contact surface 14 of a drug solution 30, in a slide-contact surface 11 of the piston member 10, slidingly making contact with an inner circumferential surface 2 of the syringe barrel 1. After cut-processing, pitch P of the protruded rims 13 is 50 µm or smaller, and press-fit margin T to the syringe barrel 1 is 10 to 150 µm.

## Description

### TECHNICAL FIELD

The present invention relates to piston members such as a gasket and a middle piston for a syringe used when administering a drug solution to the human body or an animal in fields of medicinal drugs and medicine.

### BACKGROUND ART

An injection syringe prior to having an injection needle mounted thereto includes a syringe barrel (cylindrical tube) made from glass or plastic, a movable plunger rod (plunger), a gasket that is attached at a front end portion of the plunger rod and is a piston member for maintaining non-leakage and vapor impermeability and ensuring slidability of the plunger rod, and a top cap attached to a needle mount part of the syringe barrel. Vulcanized rubber has been conventionally used for gaskets, and silicone grease has been applied to the surface of the gaskets or the inner surface of syringe barrels in order to improve inferior slidability when the gaskets made from rubber slides on the inner surface of the syringe barrels.

However, there have been problems such as reduction in potency caused when an active ingredient in a drug solution is adsorbed in the silicone grease, and contamination of the drug solution by silicon particles in the silicone grease, and adverse effect thereof to the human body. In addition, there has been a fear of elution of soluble components contained in the rubber in the drug solution. In particular, since pre-filled syringes, which are used more frequently in recent years, are filled with a drug solution in advance and stored over a long period of time to be used; gaskets of the pre-filled syringes are required to have higher performance than those for ordinary injection syringes with regard to having a quality that does not change over a long period of time, being able to be used safely, ensuring sealing performance (non-leakage and vapor impermeability) with respect to highly permeable drug solutions, and also having slidability equivalent to that of ordinary injection syringes. With regard to this point, the same applies for gaskets mounted on a middle piston and the front end of a piston rod used in a dual syringe having a syringe barrel whose front end side is filled with a powder drug and whose piston rod side is filled with injection water via the middle piston.

In order to solve such a problem, a gasket that has been developed is obtained by affixing a medical-application plug covering film formed from a polytetrafluoroethylene (hereinafter, referred to as "PTFE") film on an outer circumferential surface of a main body of the gasket where a slide-contact is made between a front end surface of a liquid contact side of the gasket main body made from rubber and an inner circumferential surface of a syringe barrel, and continuously and integrally forming a plurality of independent ring-shaped protruded rims on the outer circumferential surface adjacent to the front end surface of the liquid contact side (Patent Literature 1).

The gasket is formed by affixing, to a mold having multiple streaks of independent ring-shaped grooves formed on the inner circumference of the bottom of a cavity of the mold, a PTFE film that is formed piece by piece with a cast method, has a film thickness of about 20 to 60 µm, and whose adhesion surface to the gasket main body is surface-treated to have increased adhesive strength with rubber, press fitting the gasket main body made from rubber into the cavity to stretch the PTFE film and affix the PTFE film to the surface of the gasket main body, and lastly excising the PTFE film that has emerged out from a piston-rod mounting side end of the gasket main body. At the press fitting step, a plurality of independent ring-shaped protruded rims are formed on the front end portion of the PTFE film affixed to the surface thereof by ring-shaped grooves formed on the cavity of the mold.

The gasket can, because of having the PTFE film, largely improve slidability of the gasket with respect to the syringe when compared to a gasket made from rubber, and prevents, because of the plurality of independent ring-shaped protruded rims, leakage of drug solution loaded in the front end side of the syringe barrel in a direction from a slide-contact surface between the syringe barrel and the gasket toward the piston rod side. However, the gasket disclosed in Patent Literature 1 has problems described in the following.

### CITATION LIST

### [PATENT LITERATURE]

[PTL 1] Japanese Laid-Open Patent Publication No. 2006-181027

### SUMMARY OF THE INVENTION

### PROBLEMS TO BE SOLVED BY THE INVENTION

(1) In the gasket obtained through such a PTFE film affixing method, at an attachment portion, depending on the length and diameter of the gasket, the PTFE film is stretched about three-fold with a gasket for a small-volume syringe of 5 ml or smaller, about two-fold with a gasket for a middle-volume syringe of larger than 5 ml but not larger than 100 ml, and about 1.5-fold with a gasket for a large-volume syringe of larger than 100 ml. Generally, when a resin film such as a PTFE film is stretched, the elongation direction of the molecules in the resin becomes uniform in accordance with the stretching. Therefore, orientation of the resin film becomes high, resulting in the resin film being unable to elongate with respect to a direction perpendicular to the elongation direction, and performance as a resin gradually deteriorates, and, when the deterioration is extensive, whitens to ultimately become torn. With the PTFE film, water repellency deteriorates as the film becomes oriented due to elongation, and tearing ultimately occurs in the film at the outer circumferential portion of the front end surface on the liquid contact side of the gasket main body that has been most rigorously stretched, resulting in reduced yield rate and quality degradation as a product.
   When forming a gasket with such a press fitting method in particular, depending on the shape of the gasket, since the PTFE film is greatly stretched at the outer circumferential portion of the front end surface on the liquid contact side of the gasket main body as described above when compared to other portions, degradation of resin performance at the portion is excessive. That effect, which is although thought to be alleviated to a certain degree due to the multiple independent ring-shaped protruded rims, may not be such a problem with middle and large volume syringes but causes a large problem for practical use with small-volume syringes of 5 ml or smaller due to having a small diameter of about 6 mm. The ring-shaped protruded rims formed on the film by the press fitting method are created through transcription from the cavity. However, since the gasket main body pushed in the cavity is made of rubber, the finished shape will inevitably have a certain degree of inaccuracy. In addition, the shape may become flawed since the ring-shaped protruded rims formed on the front end of the gasket have to be forcibly pulled out from the ring-shaped grooves when pulling out the gasket from the cavity on which the ring-shaped grooves are formed. Therefore, there is a limit to the degree of the alleviation effect.
(2) In addition, fine concavities and convexities exist on the surface of the used PTFE film obtained through the cast method, and these fine concavities and convexities are stretched through the elongation described above, leading to leakage of liquid along the film surface. In addition, the PTFE film including multiple applied layers has a large number of fine through-holes therein. Since the ring-shaped protruded rims in Patent Literature 1 have a width of 0.05 to 0.5 mm, a height of 0.01 to 0.2 mm, and the gasket main body is made from rubber; the ring-shaped protruded rims are barely compressed when the gasket main body is inserted in the syringe barrel. Therefore, the fine through-holes in the ring-shaped protruded rims remain without being crushed, leading to a problem of causing leakage of liquid through the fine through-holes. The leakage of liquid through the surface and inside the ring-shaped protruded rims is particularly significant in small-volume syringes, and this leakage has been an obstacle of practical use in small-volume syringes.
(3) When the PTFE film is manufactured through a cast method, a solution having PTFE suspended therein has to be applied multiple times and sintered every time, leading to a problem of a large manufacturing cost for the PTFE film itself.
(4) In addition, when affixing the PTFE film on the gasket main body with a press fitting method, the PTFE film can only be physically affixed to a front end portion on one side of the gasket main body. Therefore, the end of the gasket main body on the opposite side becomes inevitably uncovered. A middle piston of a dual syringe which uses a powder drug cannot be produced with this method, leading to a problem of limited use application.
(5) Furthermore, with such a method, because of temperature change during heat disinfection or the press fitting process, there have been problems such as weakened adhesion between rubber forming the gasket main body inside and the thin PTFE film affixed on the surface through thermal expansion difference, and wrinkles being generated on the surface of the PTFE film.

In view of such conventional examples, a main objective of the present invention is to provide a low cost piston member for a syringe, having sufficiently satisfying slidability and sealing performance such as non-leakage and vapor impermeability, without being limited to size, from small diameters to large diameters.

### SOLUTION TO THE PROBLEMS

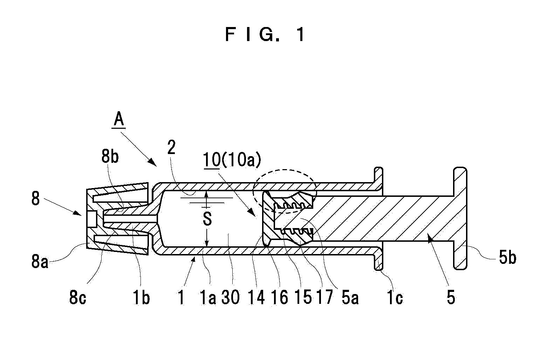

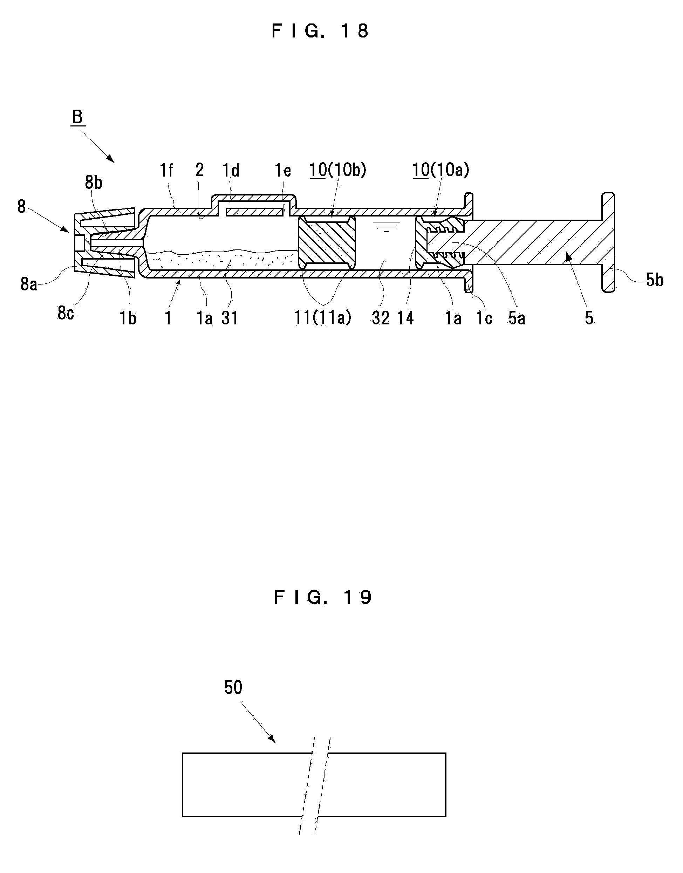

An invention disclosed in claim 1 is a piston member 10 that is a gasket 10a (FIG. 1) or a middle piston 10b (FIG. 18) used in a syringe barrel 1.

The piston member 10 formed by cut-processing a PTFE block and pressed and fitted in a syringe barrel 1 to be used in a slidable manner,
the piston member including protruded rims 13 formed at least in a circumferential direction of a whole circumference of a slide-contact surface 11a that is adjacent to a liquid contact surface 14 in contact with a drug solution 30, and that is a part of a slide-contact surface 11 of the piston member 10, slidingly making contact with an inner circumferential surface 2 of the syringe barrel 1, wherein
after the cut-processing, a pitch P of the protruded rims 13 is not larger than 50 µm, and a press-fit margin T of the piston member 10 with respect to the syringe barrel 1 is 10 to 150 µm.

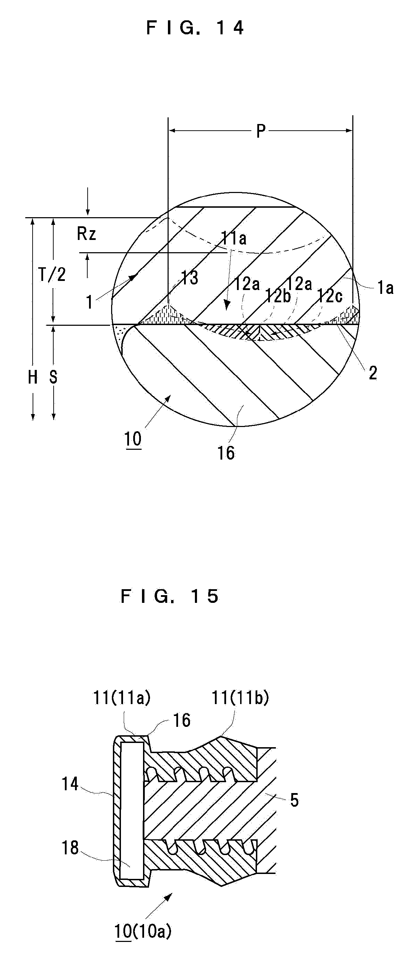

The PTFE block is cut-processed (ordinarily, through lathing) by taking into consideration the press-fit margin T in accordance with an internal diameter S of the syringe barrel 1, and the protruded rims 13 are formed on the cut-processed surface (particularly on the slide-contact surface 11a in contact with the syringe barrel 1) such that, when being cut-processed, the pitch P is not larger than 50 µm (preferably 3 to 40 µm) and the diameter difference of the press-fit margin T of the piston member 10 with respect to the syringe barrel 1 is 10 to 150 µm. When the piston member 10 formed in such manner is inserted in the syringe barrel 1, the protruded rims 13 of the piston member 10, which has been pressed and fitted in, are crushed by the inner circumferential surface 2 of the syringe barrel 1 and cold-flow toward processed grooves 12, and the cold-flowing buries the processed grooves 12 (FIG. 14). As a result, a high level of water tightness (i.e., non-leakage and vapor impermeability) is achieved when the piston member 10 is inserted in the syringe barrel 1. When the press-fit margin T is 10 to 150 µm, slide resistance of the piston member 10 with respect to the syringe barrel 1 during injection becomes the required level of 12 N or less.

In the invention according to claim 2 based on the piston member 10 in claim 1, a maximum height roughness Rz after the cut-processing is set to be not larger than 6 µm. When the maximum height roughness (in other words, groove depth or 10-point average roughness) Rz of the processed grooves 12 is not larger than 6 µm (preferably not larger than 3 µm), burial of the processed grooves 12 through the cold-flowing is performed with certainty.

In the invention according to claim 3 based on the piston member 10 of claim 1, the protruded rims 13 are helically formed. When the processed grooves 12 are helical grooves, although it may be thought in common sense that the drug solution 30 will flow out along the helical processed grooves 12; a high level of water tightness is achieved since the cold-flowing buries the processed grooves 12 as described above. When the processed grooves 12 are helical, the piston member 10 can be formed with ordinary lathing, and manufacturing can be performed rapidly at low cost.

In the invention according to claim 4 based on the piston member 10 of claim 1, the protruded rims 13 are formed in a ring shape. In this case, since the pitch P of the protruded rims 13 is very small as 50 µm and thereby the thicknesses of the protruded rims 13 themselves are very small, the placebo 30 leaks within the protruded rims 13 by passing through a grain boundary of a PTFE lump existing in the protruded rims 13. However, by setting the press-fit margin T as 10 to 150 µm, the grain boundary of the PTFE lump is resolved when the protruded rims 13 are pressed and crushed by being press-fitted to the syringe barrel 1 as described above, and not only leakage of liquid from the grain boundary is blocked but also passing of vapor is blocked. This also applies to the case with a helical shape. It should be noted that the slide resistance here is also 12 N or less, similarly.

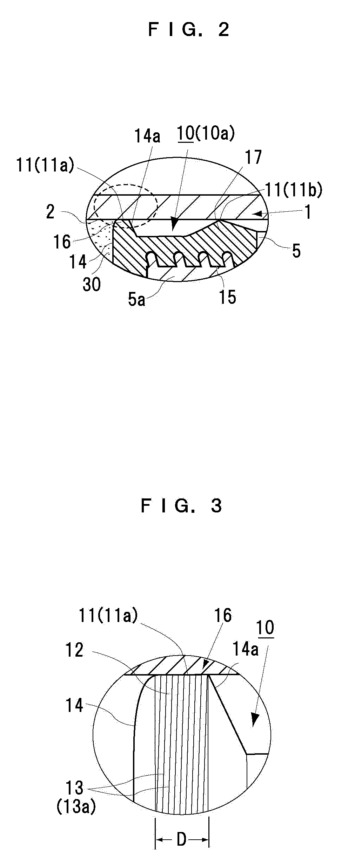

In the invention (cf. FIG. 2) according to claim 5 based on the piston member 10 according to any one of claims 1 to 4, a width D of the slide-contact surface 11a of the piston member 10 is 0.5 mm to 3 mm. When the width D of the slide-contact surface 11a is not larger than 0.5 mm, a liquid contact-side sliding part 16 on which the slide-contact surface 11 a is formed becomes weak in terms of strength and may break during handling. When the width D is not smaller than 3 mm and when the syringe barrel 1 is a cycloolefin resin, a surface pressure of the press-fitted liquid contact-side sliding part 16 with respect to the syringe barrel 1 becomes too high, and the syringe barrel 1 may crack.

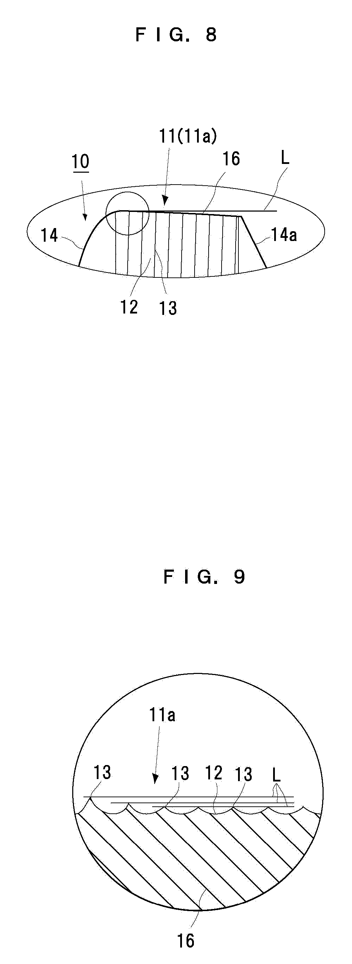

In the invention (cf. FIGS. 8 and 9) according to claim 6 based on the piston member 10 according to any one of claims 1 to 4, the slide-contact surface 11a adjacent to the liquid contact surface of the piston member 10 is formed to be gradually smaller from a side of the liquid contact surface 14 to a side of a liquid non-contact surface 14a opposite thereto.

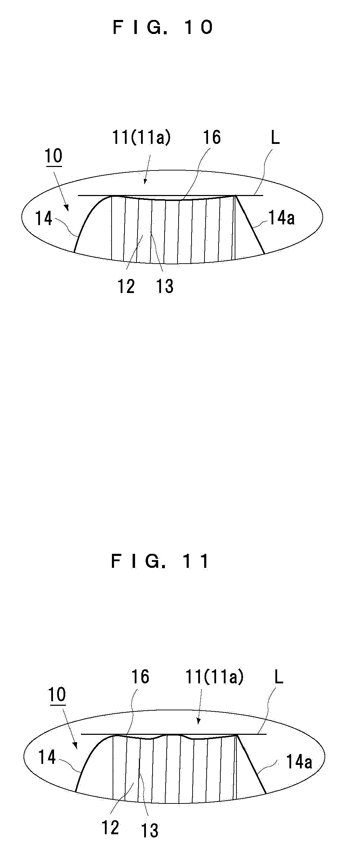

In the invention (cf. FIG. 10) according to claim 7 based on the piston member 10 according to any one of claims 1 to 4, the slide-contact surface 11a adjacent to the liquid contact surface of the piston member 10 is formed to be small, in a concaved arc shape, between a side of the liquid contact surface 14 and a side of a liquid non-contact surface 14a opposite thereto.

In the invention (cf. FIG. 11) according to claim 8 based on the piston member 10 according to any one of claims 1 to 4, the slide-contact surface 11a adjacent to the liquid contact surface of the piston member 10 is formed to be small, in concaved arc shapes, between a central portion of the liquid contact surface 14 and a liquid non-contact surface 14a opposite thereto, and both of the surfaces.

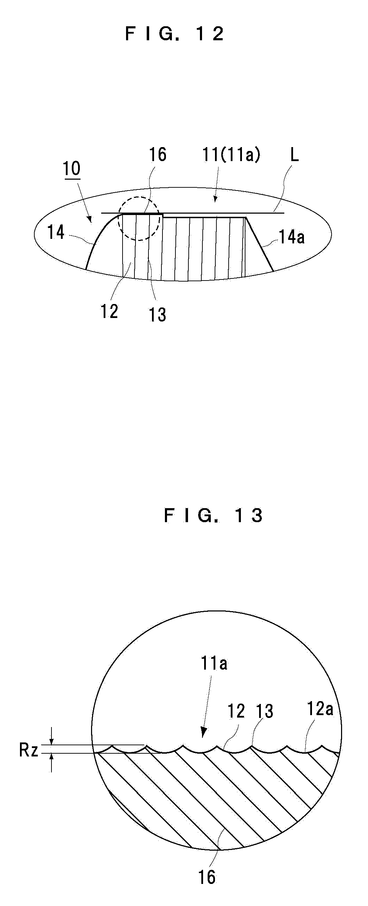

In the invention (cf. FIG. 12) according to claim 9 based on the piston member 10 according to any one of claims 1 to 4, a diameter of the piston member 10 at the slide-contact surface 11a adjacent to the liquid contact surface is set to be larger on a side of the liquid contact surface 14 than a side of a liquid non-contact surface 14a opposite thereto.

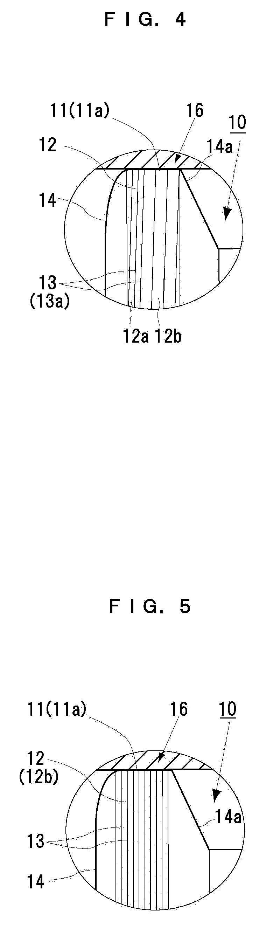

In the invention (cf. FIG. 4) according to claim 10 based on the piston member 10 according to any one of claims 1 to 4, at least three of the protruded rims 13 (in other words, three full circumferences) counted from the liquid contact surface 14 have the pitch P, the maximum height roughness Rz, and the press-fit margin T set forth in claim 1, and the protruded rims 13 subsequent to the fourth rim (in other words, the fourth full circumference) have at least one of the pitch P, the maximum height roughness Rz, and the press-fit margin T set to be larger than that of the three protruded rims 13 on the side of the liquid contact surface 14.

By adopting such a configuration, contact pressure by the syringe barrel 1 on the inner circumferential surface 2 is increased on the liquid contact surface 14 side to enhance reliability against leakage of liquid, and the contact pressure is reduced on the syringe barrel 1 at the narrow portion or the portion narrowed into a concaved arc shape to allow pressing of a piston rod 5 with lesser force.

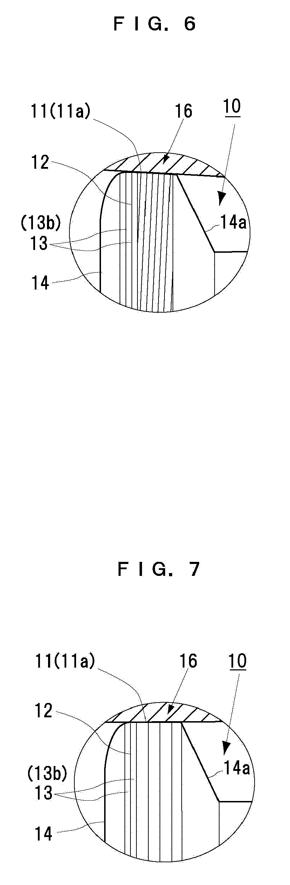

In the invention (cf. FIG. 6) according to claim 11 based on the piston member 10 according to any one of claims 1 to 4, at least three of the protruded rims 13 (in other words, 3 full circumferences) counted from the liquid contact surface 14 have a ring shape, and the protruded rims 13 subsequent to the fourth rim (in other words, the fourth full circumference) are helical.

In this case, water tightness is ensured by the protruded rims 13 (at least 3 rims up to 5 rims) formed along the narrow processed grooves 12 on the liquid contact surface 14 side, and processing speed can be significantly improved by forming the processed grooves 12 that do not contribute to ensure water tightness on the liquid non-contact surface 14a side to be helical.

In the invention (cf. FIGS. 2 and 19) according to claim 12 based on the piston member 10 according to any one of claims 1 to 4, the whole piston member 10 is formed from PTFE. In this case, the piston member 10 can be produced cheaply and rapidly through the same procedure as ordinary machining by only cut-processing a PTFE bar material 50.

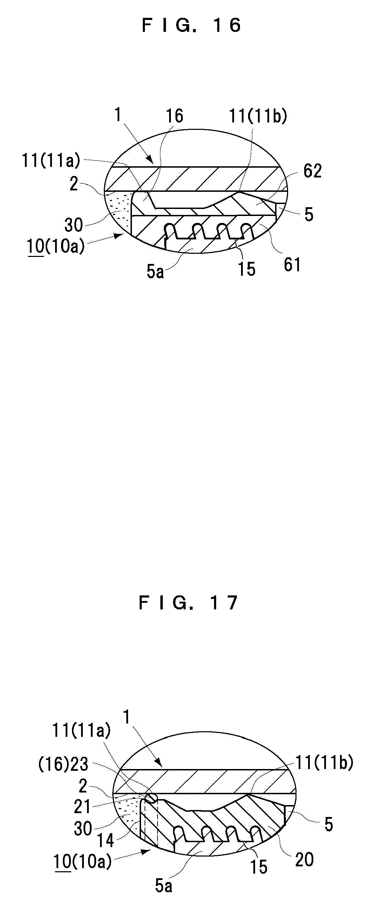

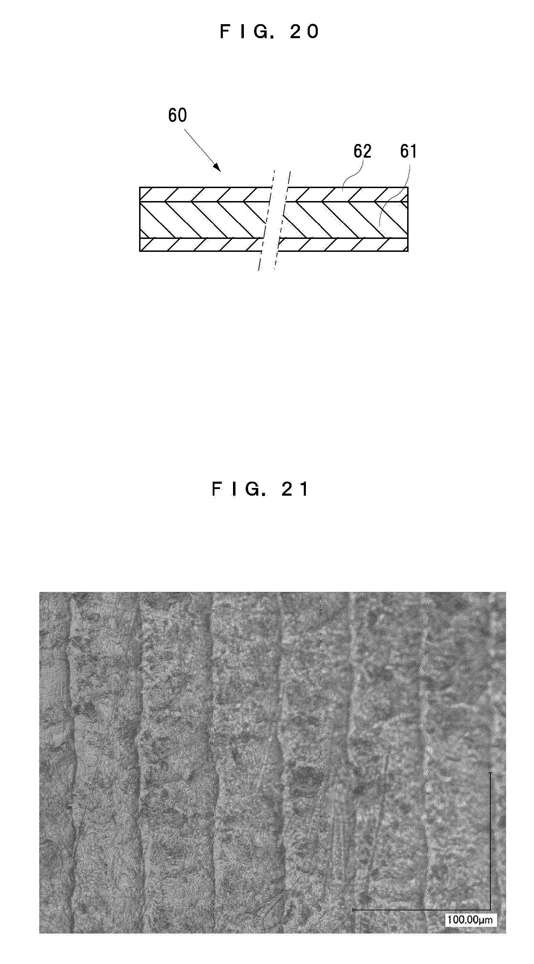

In the invention (cf. FIGS. 16 and 20) according to claim 13 based on the piston member 10 according to any one of claims 1 to 4, the piston member 10 is formed by cut-processing at least a PTFE portion of a composite block 60 in which only an outer circumferential surface of a drug solution-resistant resin material 61 is covered with a PTFE cylindrical material 62. In this case, usage of expensive PTFE can be reduced since the composite block 60 is used. In addition, smoothness of the manufactured piston member 10 is ensured by the soft PTFE, and strength and thermal expansion of the piston member 10 are governed by the drug solution-resistant resin material 61. In other words, the piston member 10 is almost not subjected to the influence of the PTFE that shows large thermal expansion around room temperature, and thereby pre-filled syringes A and B having incorporated therein the piston member 10 formed from the composite block 60 are not subjected to thermal effect of usage environment.

In the invention (cf. FIG. 15) according to claim 14 based on the piston member 10 according to any one of claims 1 to 4, a cavity 18 is formed inside the slide-contact surface 11a of the piston member 10. In this case, since the cavity 18 is formed inside the slide-contact surface 11a, the slide-contact portion has a small thickness and elasticity improves. As a result, touch by the syringe barrel 1 to the inner circumferential surface 2 becomes softer, and it becomes possible to press the piston rod 5 with lesser force.

### ADVANTAGEOUS EFFECTS OF THE INVENTION

With the present invention, it is possible to provide a low cost piston member for a dual syringe or a pre-filled syringe, having sufficiently satisfying slidability and water tightness such as vapor impermeability and leakage-less property, without being limited to size, from small diameters to large diameters, and without using a medical-application plug covering film or silicone oil.

### BRIEF DESCRIPTION OF THE DRAWINGS

[FIG. 1] FIG. 1 is a cross sectional view of a pre-filled syringe in which the present invention is applied.
[FIG. 2] FIG. 2 is an enlarged cross sectional view of a portion shown with an ellipse drawn by broken line in FIG. 1.
[FIG. 3] FIG. 3 is an enlarged front view in which processed grooves in a portion shown in an ellipse drawn by broken line in FIG. 2 are helical.
[FIG. 4] FIG. 4 is a front view of a modification of the helical processed grooves in FIG. 2.
[FIG. 5] FIG. 5 is an enlarged front view in which the processed grooves in the portion shown in the ellipse drawn by broken line in FIG. 2 have a ring shape.
[FIG. 6] FIG. 6 is an enlarged front view in which processed grooves have ring shapes on a liquid contact surface side and helical shapes on the opposite side in the portion shown with the ellipse drawn by broken line in FIG. 2.
[FIG. 7] FIG. 7 is an enlarged front view in which the processed grooves have narrow ring shapes on the liquid contact surface side and wide ring shapes or helical shapes on the opposite side in the portion shown with the ellipse drawn by broken line in FIG. 2.
[FIG. 8] FIG. 8 is an enlarged cross sectional view in which a diameter at the slide-contact surface is gradually reduced from the liquid contact side toward a piston rod side in the portion shown with the ellipse drawn by broken line in FIG. 2.
[FIG. 9] FIG. 9 is an enlarged cross sectional view in which protruded rims are formed such that peak diameters thereof are gradually reduced from the liquid contact side toward the piston rod side in the portion shown with a circle in FIG. 8.
[FIG. 10] FIG. 10 is an enlarged cross sectional view in which slide-contact surface is formed to have a concaved arc shape between the liquid contact side and the piston rod side in the portion shown with the ellipse drawn by broken line in FIG. 2.
[FIG. 11] FIG. 11 is an enlarged cross sectional view in which the slide-contact surface is formed to have concaved arc shapes between a mid-portion of the liquid contact side and the piston rod side, and both surfaces thereof in the portion shown with the ellipse drawn by broken line in FIG. 2.
[FIG. 12] FIG. 12 is an enlarged cross sectional view in which the liquid contact side of the slide-contact surface is formed to be thicker than the piston rod side in the portion shown with the ellipse drawn by broken line in FIG. 2.
[FIG. 13] FIG. 13 is an enlarged cross sectional view of a portion shown with an ellipse drawn by broken line in FIG. 12.
[FIG. 14] FIG. 14 is an enlarged cross sectional view when a piston member is press-fitted in a syringe barrel.
[FIG. 15] FIG. 15 is an enlarged cross sectional view in which a cavity is formed inside the slide-contact surface of a gasket in FIG. 1.
[FIG. 16] FIG. 16 is an expanded sectional view of relevant parts of a gasket formed using a composite block.
[FIG. 17] FIG. 17 is an expanded sectional view of relevant parts of a gasket formed using a PTFE ring.
[FIG. 18] FIG. 18 is a cross sectional view of a dual syringe in which the piston member of the present invention is used.
[FIG. 19] FIG. 19 is a front view of a PTFE sintered body used in present invention.
[FIG. 20] FIG. 20 is a cross sectional view of the composite block used in the present invention.
[FIG. 21] FIG. 21 is an inverted 1000x magnification picture of a cut surface of the gasket of the present invention.
[FIG. 22] FIG. 22 is an inverted 1000x magnification picture when the cut surface of the gasket of the present invention is viewed diagonally.
[FIG. 23] FIG. 23 shows the results of vapor permeability test performed on the gasket of the present invention.
[FIG. 24] FIG. 24 shows the results of slide resistance measurement performed on the gasket of the present invention.
[FIG. 25] FIG. 25 shows the results of roughness measurement (pitch = 50 µm) of the surface of the gasket of the present invention.
[FIG. 26] FIG. 26 shows the results of roughness measurement (pitch = 100 µm) of the surface of the gasket of the present invention.
[FIG. 27] FIG. 27 is an enlarged picture showing a contact state of a liquid contact-side sliding part to the inner surface of the syringe barrel in the gasket of the present invention.

### DESCRIPTION OF EMBODIMENTS

In the following, the present invention will be described in accordance with illustrated examples. FIG. 1 is a cross sectional view of a pre-filled syringe A in which a gasket 10a of the present invention is applied. FIG. 18 is a cross sectional view of a dual syringe B in which the gasket 10a and a middle piston 10b of the present invention are applied. As shown in FIG. 1, the pre-filled syringe A includes the gasket 10a which is a piston member 10, a syringe barrel 1, a piston rod 5 mounted on the gasket 10a, and a top cap 8. In the dual syringe B, the middle piston 10b which is the piston member 10 is additionally used. In FIG. 1, reference character 30 represents a drug solution (injection) loaded in the syringe barrel 1. In FIG. 18, reference character 31 represents a powder drug, and reference character 32 represents injection water for dissolving the drug. In the following, FIG. 1 will be described. It should be noted that, in the present specification, common members are indicated with the same reference character. In FIG. 2 and further, the description of overlapping portions that already have been provided are to be cited and description thereof is omitted in principle to prevent the description from being complicated.

The syringe barrel 1 is a cylindrical container. A mount part 1b on which an injection needle that is not shown is mounted is disposed at the front end of a barrel main body 1a in a protruding manner, and a flange 1c for finger placement is formed on the back end of the barrel main body 1a. For the material of the syringe barrel 1, glass, hard resin (e.g., cycloolefin resin which is hereinafter referred to as "COP"), or flexible resin (e.g., polypropylene which is hereinafter referred to as "PP") is used.

Two types exist for the piston member 10 including the gasket 10a shown in FIG. 1 and the middle piston 10b shown in FIGS. 16 and 17: one whose entirety is formed from PTFE, and one whose central portion is formed from a drug solution-resistant resin 61 and whose portion that is to be cut is at least formed from PTFE. Although the PTFE used in the present invention may be pure PTFE, it is more preferable to use, since the piston member 10 becomes elastic, a modified object having mixed therein, for example, 1 to 15 mass% of a fluorine resin such as a tetrafluoroethylene-hexafluoropropylene copolymer, and a polytetrafluoroethylene - perfluoroalkyl vinyl ether copolymer (abbreviated name: PFA) which are crystallization inhibitors of PTFE. Around bar material 50 as shown in FIG. 19 is used for the PTFE before processing.

As the PTFE used in the present invention, pure PTFE, the modified objects of PTFE described above, or a block (round bar material) closed-celled by a hot isostatic pressing method that is called HIP treatment is used. The PTFE before the HIP treatment is powder PTFE or one that is obtained by forming the modified PTFE into a block shape (e.g., columnar) with high pressure and sintering thereof. Therefore, the PTFE before the HIP treatment is an aggregate of fine PTFE lumps and is an open cell type block in which fine gaps are continuously connected at grain boundaries of the PTFE lump.

One example of how the hot isostatic pressing method is performed is shown next. The open cell type PTFE block or the open cell type modified PTFE block (e.g., rod-like body) is placed in a heating furnace, and the pressure in the heating furnace first is reduced to a degree of vacuum of 0.013 to 133 Pa (a commonly used degree of vacuum is 0.13 to 13.3 Pa) to remove gas from the pressure formed body. Then, the closed cell type PTFE block is obtained by performing thermal-fusion at 320 to 400°C (more preferably at 350 to 370°C) for several to less than twenty hours while maintaining the reduced pressure state, and cooling thereof. Alternatively, the closed cell type PTFE block is obtained similarly by increasing the temperature to the above described temperature under the pressure reduced to the degree of vacuum described above, maintaining the temperature for the above described time, returning the pressure to ordinary pressure, and cooling thereof under pressure.

When the hot isostatic pressing method is applied to an open cell type PTFE block, gas is pulled out from gaps (space) between grain lumps of the PTFE forming the PTFE block, and the gaps (space) are reduced through the rapid expansion, caused by the hot isostatic pressing, of the contact interfaces of the grain lumps softened by the heating. At this moment, gas does not exist in the gaps (space) to provide resistance and can be reduced to a minimum, and open cells that have existed before the heating dissipate.

FIG. 20 shows a composite block 60 in which the outer circumferential surface of the round rod-shaped drug solution-resistant resin material 61 is covered with a PTFE cylindrical material 62 that is to be cut-processed. Examples of the drug solution-resistant resin material 61 include COP and ultrahigh molecular weight polyethylene. The outer circumferential surface of the drug solution-resistant resin material 61 is covered by inserting the drug solution-resistant resin material 61 into the cylindrical PTFE member 62 that has been heated, and cooling and shrinking the cylindrical PTFE member 62 to perform so-called shrink-fitting. As another method, the round bar material of the drug solution-resistant resin material 61 may be press-fitted and integrated with the cylindrical PTFE member 62. Integration is achieved by fastening the PTFE member 62 that has extended through the press-fitting. In this case, it is sufficient when the PTFE member 62 has a thickness (1 to 2 mm) necessary for cut-processing. When the diameter of the drug solution-resistant resin material 61 is sufficiently larger with respect to the thickness of the PTFE member 62, influence of PTFE that shows large thermal expansion around room temperature is negated, the composite block 60 is governed by thermal expansion of the drug solution-resistant resin material 61 which is smaller than thermal expansion of PTFE, and dimensional change of the composite block 60 is sufficiently suppressed in temperatures from around 0°C to around room temperature. The PTFE member 62 of the composite block 60 may be the open cell type described above or may be turned into the closed cell type through the HIP treatment.

Next, the shape of the gasket 10a in FIG. 1 will be briefly described. In the gasket 10a, outer circumferences of a liquid contact-side sliding part 16 and an end part 17 on the mounting side of the piston rod 5 are formed to be large, and the outer circumferences at parts between the liquid contact-side sliding part 16 and the end part 17 on the mounting side are formed to be small. The end part 17 guides slide-movement of the gasket 10a such that the piston rod 5 mounted on the gasket 10a does not wobble, and the maximum external diameter thereof is set to be almost equal to or slightly smaller than the internal diameter of the syringe barrel 1. The shape of the outer circumferential portion of the end part 17 is formed such that its cross section that is parallel to the central axis has a mound shape, and the outer circumference thereof slidingly makes contact with the inner circumference of the syringe barrel 1. However, the end part 17 may be omitted if there is no adverse effect on slide-movement of the piston rod 5.

On the other hand, the liquid contact-side sliding part 16 of the gasket 10a is a slide-contact portion that slidingly makes contact with an inner circumferential surface 2 of the syringe barrel 1, and is adjacent to a liquid contact surface 14 where a contact with the drug solution 30 is made, and the liquid contact-side sliding part 16 has a certain width D from the liquid contact surface 14 to the end part 17 on the mounting side of the piston rod 5. On the whole circumference of a slide-contact surface 11a thereof, processed grooves 12 are formed in the circumferential direction. The width D is 0.5 to 3 mm. The width D is preferably 1 to 2 mm. The width D is applied in examples with the helical shape, the independent ring shapes, and in modifications thereof. The processed grooves 12 may be helical or may have independent ring shapes as shown respectively in FIGS. 3 and 5. Detailed description thereof will be provided later.

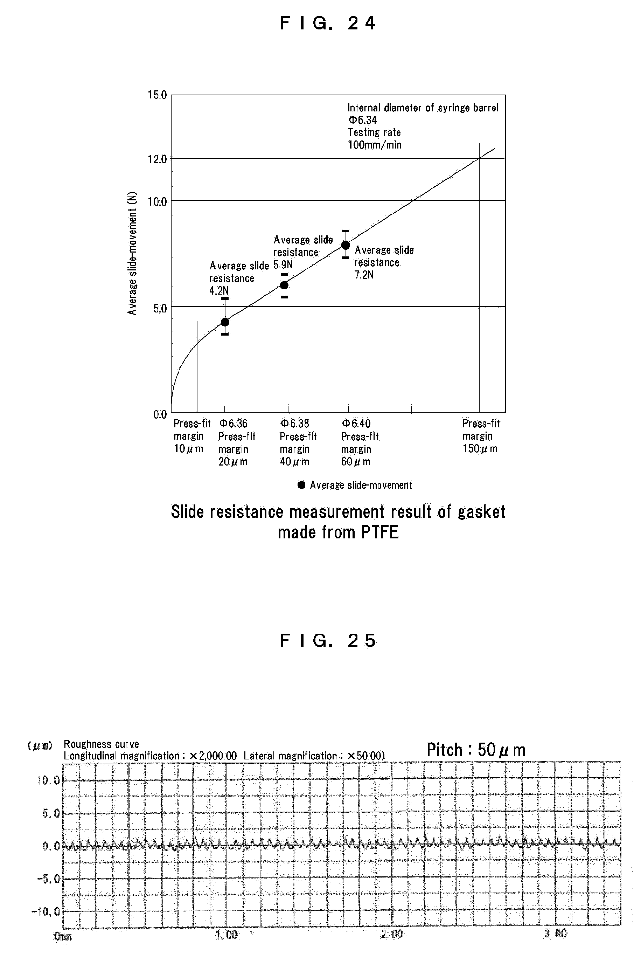

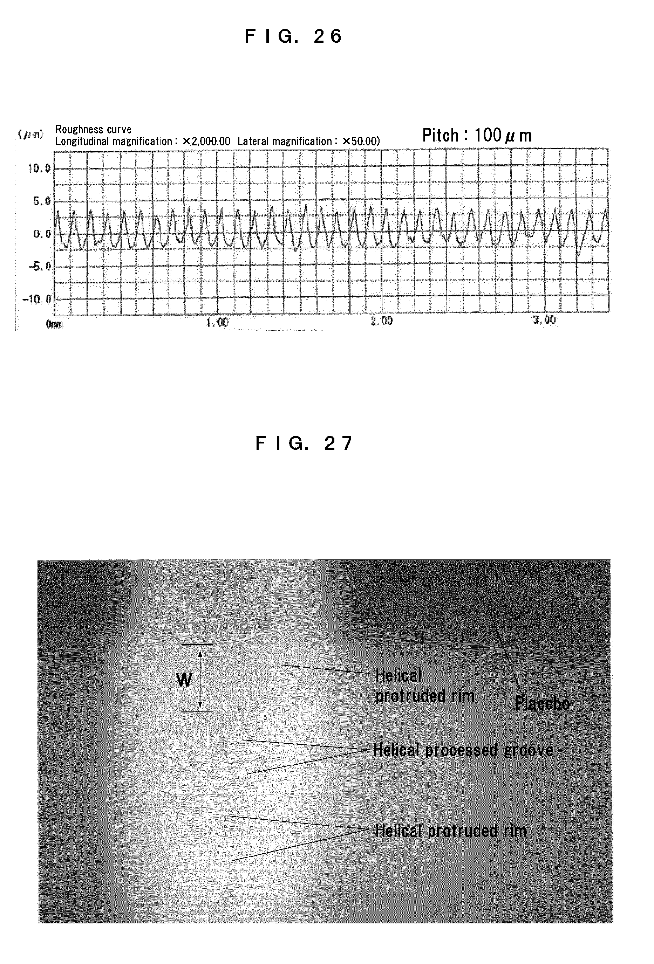

As a processing method, ordinarily, cut-processing with a lathe is selected in consideration of the shape of the piston member 10. Examples of a cutting tool (bit) used in the cut-processing include high speed steel, cemented carbide, and polycrystal and monocrystal diamonds capable of smoothly shaving the surface of PTFE to a certain degree. FIG. 13 is a partially-expanded sectional view of a cut portion, and protruded rims 13 whose cross sections are approximately triangular are formed between the processed grooves 12 shaved by the cutting tool. Here, the height from a groove bottom 12a of the processed grooves 12 to the top part of the protruded rims 13 is a maximum height roughness Rz (i.e., 10-point average roughness, more specifically, groove depth). Actual measurement data are shown in FIGS. 25 and 26. FIG. 25 shows a case when a pitch P of the protruded rims 13 was 50 µm and the maximum height roughness Rz was 2.3 µm (an arithmetical mean roughness Ra here was 0.36 µm), and leakage of the placebo was not observed in a later described leakage test. FIG. 26 shows a case in which the pitch P of the protruded rims 13 was 100 µm and the maximum height roughness Rz was 6.6 µm (the arithmetical mean roughness Ra here was 1.54 µm), and leakage of the placebo was observed in the later described leakage test. It should be noted that a surface shape analyzing device manufactured by Tokyo Seimitsu (K.K.) was used for the measurement.

The cutting method is performed by projecting the PTFE block 50 or 60 (bar material, preferably a round bar) beyond a chuck of the lathe by a predetermined amount, shaving off the end surface of the projected portion flatly or in a predetermined required shape, shaving the outer circumferential surface of the projected portion in a predetermined shape described later depending on whether the piston member 10 is the gasket 10a or the middle piston 10b. In the case with the middle piston 10b, after cutting of the external outer shape is finished, the base of the projected portion is cut across to form the middle piston 10b. In the case with the gasket 10a, a tap drill hole of a female-screw hole 15 for screwing in a male-screw part 5a of the piston rod 5 is additionally bored at the center of the end surface of the projected portion, a female-screw thread is engraved on the tap drill hole through tapping, opening edges of the female-screw hole 15 are chamfered, and lastly the base of the projected portion is cut across in a predetermined dimension to form the gasket 10a. The feed width is indicated with the pitch P in FIG. 14.

In the cut-processing described above for cutting the slide-contact surface 11a of the piston member 10, in the case with helical protruded rims 13a in FIG. 3, the helical processed grooves 12 are formed so as to have the later described pitch P (not larger than 50 µm) by moving, at a constant speed, the bit over the whole slide-contact surface 11a. FIG. 4 shows a modification thereof, and only a portion (e.g., 3 to 5 rims for the helical grooves (i.e., 3 to 5 full circumferences)) adjacent to the liquid contact surface 14 is cut to have a pitch not larger than 50 µm, and other portions are cut to have a wider pitch to increase the cutting speed.

FIGS. 5 to 12 show modifications of the slide-contact surface 11a of the piston member 10. FIG. 5 shows a case in which ring-shaped protruded rims 13b are formed over the whole slide-contact surface 11a at equal intervals. The cutting tool is brought in contact with the PTFE round bar material 50 to shave off a single pitch P of the processed grooves 12, the cutting tool is retreated and separated from the PTFE round bar material 50, the cutting tool is horizontally moved by a single pitch P, and the cutting tool is brought in contact with the PTFE round bar material 50 again to shave off a single pitch P of the processed grooves 12. This operation is repeated to form the ring-shaped protruded rims 13b on the slide-contact surface 11a.

FIG. 6 shows a case in which a single rim (i.e., a full circumference) of the protruded rims 13 is formed on the liquid contact surface 14 side, 3 to 5 rims (i.e., 3 to 5 full circumferences) of the ring-shaped protruded rims 13b are additionally formed as insurance at the pitch P of not larger than 50 µm, and the helical protruded rims 13a are formed in the remaining portions. In FIG. 7, a single rim of the protruded rims 13 is formed on the liquid contact surface 14 side and 3 to 5 rims of the ring-shaped protruded rims 13b are additionally formed as insurance at the pitch P of not larger than 50 µm in a similar manner, but the ring-shaped protruded rims 13b having a wide pitch of not smaller than 50 µm are formed in the remaining portions. As described above, FIGS. 3 to 7 show examples of creating the protruded rims 13.

FIGS. 8 to 12 show modifications of the slide-contact surface 11a applied to those in FIGS. 3 to 7. Furthermore, "L" in the figures indicates a line parallel to the center line, and is provided to easily understand the shape of the slide-contact surface 11a. In FIG 8, the diameter (peak diameter H) of the liquid contact-side sliding part 16 gradually becomes smaller from the liquid contact surface 14 side to a side of a liquid non-contact surface 14a opposite thereto. FIG. 9 is a partially-expanded sectional view of a circled portion in FIG. 8. In FIG. 10, the diameter (peak diameter H) of the liquid contact-side sliding part 16 is reduced so as to form a concaved arc shape between the liquid contact surface 14 side and the liquid non-contact surface 14a side opposite thereto. In FIG. 11, the diameter (peak diameter H) of that same portion is reduced so as to form concaved are shapes between the central portion of the liquid contact surface 14 and the liquid non-contact surface 14a, and both of the surfaces. In FIG. 12, the diameter (peak diameter H) of the same portion is increased at a portion adjacent to the liquid contact surface 14, but the diameter (peak diameter H) is reduced on the liquid non-contact surface 14a side. In the piston member 10 according to the present invention, at the portion adjacent to the liquid contact surface 14, a width of at least 3 rims, and about 5 rims when ensuring water tightness and vapor impermeability, of the helical protruded rims 13a are necessary. Although a single rim of the ring-shaped protruded rims 13b is theoretically sufficient, it is preferable to have 3 rims or more as insurance. In each of the FIGS. 8 to 12, the portion having a large diameter on the liquid contact surface 14 side strongly makes contact to the inner circumferential surface 2 of the syringe barrel 1 to ensure water tightness and vapor impermeability. This is illustrated in FIG. 14. In addition, the portion that is narrowly formed gently makes contact with the inner circumferential surface 2 of the syringe barrel 1 during insertion. By adopting such a configuration, contact pressure by the syringe barrel 1 on the inner circumferential surface 2 is increased on the liquid contact surface 14 side to enhance reliability against leakage of liquid, and the contact pressure is reduced at the portion that gradually narrows or the portion narrowed into a concaved are shape to allow pressing of the piston rod 5 with lesser force. Although the portion having a large diameter is formed on the liquid contact surface 14 side in the cases described above, the present invention is not limited thereto and the portion having a large diameter may be formed at any portion of the slide-contact surface 11 a.

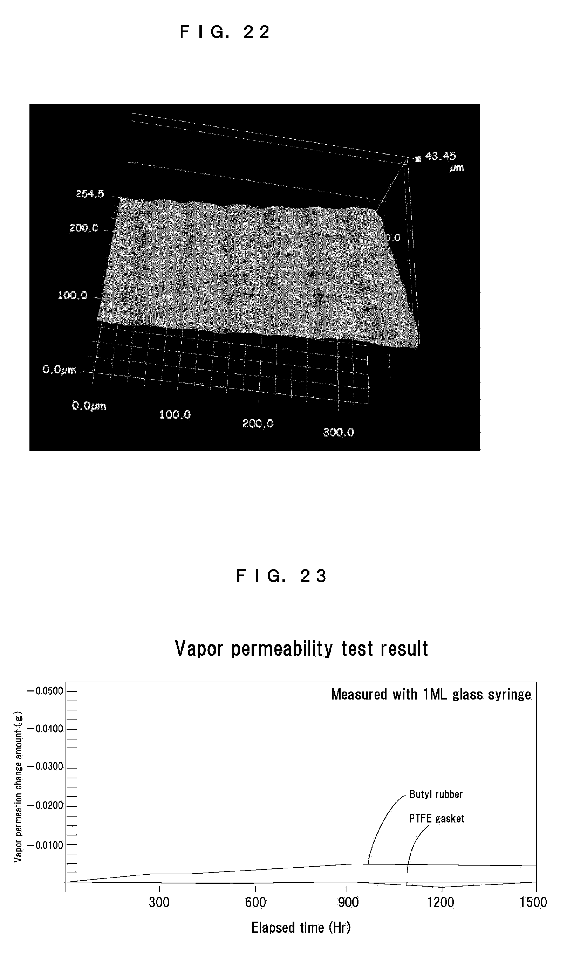

FIG. 14 shows an enlarged cross sectional view of a portion that strongly makes contact with the syringe barrel 1 described above. FIG. 21 is an inverted picture (1000x magnification) of the processed grooves 12 and FIG. 22 is an inverted picture (1000x magnification) viewing the same diagonally. FIG. 27 is an enlarged picture of a boundary portion between the placebo 30 and the liquid contact surface 14 of the piston member 10 against the inner circumferential surface 2 of the syringe barrel 1, and shows a state in which the placebo 30 is prevented from leaking by the protruded rims 13. In the picture, white-glowing lateral stripe portions are the processed grooves 12, and a state of cold flow from the protruded rims 13 not sufficiently burying the processed grooves 12 between the protruded rims 13 is shown. The protruded rims 13 in the vicinity (a range indicated by an arrow W) of a boundary portion of the liquid contact surface 14 of the piston member 10 with respect to the placebo 30 are crushed and sufficiently bury the processed grooves 12. It should be noted that, according to FIGS. 21 and 22 representing the protruded rims 13 in an inverted state, the groove bottoms 12a of the processed grooves 12 shaved off by a sharp edge of the bit form shallow continuous concaved arcs. The protruded rims 13, whose ridgelines at the tip formed on both sides of the groove bottom 12a are thin, are continuously formed as waveform bulges. However, as can be understood from FIG. 22, unlike cutting a metal, certain degree of concavities and convexities are formed on the cut-processed grooves 12 and the protruded rims 13. The cut surface is formed most roughly with high speed steel, and becomes smoother therefrom in an order of cemented carbide, polycrystal diamond, and monocrystal diamond. A smoother cut surface provides improved water tightness and steam impermeability.

As described above, certain degree of concavities and convexities are formed on the cut-processed grooves 12 and the protruded rims 13. However, PTFE is highly deformable, and when the piston member 10 is inserted in the syringe barrel 1 with a press-fit margin T, the protruded rims 13 are strongly crushed on the inner circumferential surface 2 of the syringe barrel 1 and flow (cold flow) toward the processed grooves 12, not only in the case with the helical shape but also with the ring shape. The processed grooves 12 become completely buried at most portions when the protruded rims 13 are formed to have the pitch P of not larger than 50 µm, the maximum height roughness Rz of not larger than 6 µm, and a diameter of the press-fit margin T of not smaller than 10 µm, after the cut-processing. The buried portions are shown in FIG. 14 with reference character 12a, and boundaries thereof are shown with reference character 12b. The totally buried state of the processed grooves 12 is prominent at ranges adjacent to the liquid contact surface 14 shown with the arrow W in FIG. 27. As a result, even when the processed grooves 12 are helical grooves, the above described water tightness and vapor impermeability are ensured.

When the pitch P of the protruded rims 13 is not smaller than 50 µm, depending on its relationship with the maximum height roughness Rz, sometimes the processed grooves 12 are not sufficiently buried and leakage occurs through the remaining fine gaps. In particular, when the difference in temperature of the surrounding area is particularly large (e.g., when an injection kept in a refrigerator is taken out and restored to normal temperature to be used), leakage sometimes occurs due to the physical property (of causing large dimensional change around room temperature) of PTFE. When the pitch P is not larger than 40 µm, leakage does not occur even when the difference in temperature of the surrounding area is large. A range from 3 to 40 µm provides the highest reliability in terms of non-leakage.

When the pitch P of the protruded rims 13 is not larger than 3 µm, the time required for the cut-processing becomes too long, the height of the protruded rims 13 between the processed grooves 12 becomes too low (i.e., the maximum height roughness Rz of the processed grooves 12 becomes too small), and elasticity of the protruded rims 13 becomes too small. Therefore, the pitch P of the protruded rims 13 is preferably, for practical use, not smaller than 3 µm but not larger than 40 µm.

Furthermore, when the maximum height roughness Rz of the processed grooves 12 after cutting is not smaller than 6 µm, there may be places where the PTFE that had cold-flowed does not sufficiently bury the groove bottom 12a of the processed grooves 12, and leakage may occur at those places. In terms of safety, the maximum height roughness Rz is preferably not larger than 3 µm. From a standpoint of burying the grooves with cold flow, a smaller maximum height roughness Rz is preferable. The groove bottom 12a is preferably formed in a circular arc shape for smoothly allowing the cold flow of PTFE. The pitch P and the maximum height roughness Rz of the grooves are basically independent of the thickness of the syringe barrel 1, and are applicable to the syringe barrel 1 with various thicknesses. When the pitch P and the maximum height roughness Rz of the grooves are in the above described range, high water tightness (non-leakage and vapor impermeability) is achieved even with the processed grooves 12 that are helical grooves.

Next, a relationship of the processed grooves 12, the diameter (peak diameter) H of the liquid contact-side sliding part 16 of the piston member 10, and an internal diameter S of the syringe barrel 1 will be described. With respect to the internal diameter S of the syringe barrel 1, the diameter of the liquid contact-side sliding part 16 is set to be larger by, as the press-fit margin T, at minimum 10 µm (5 µm in radius) and at maximum 150 µm (75 µm in radius) (cf. FIG. 24). As described later, when the press-fit margin T is larger than 150 µm, the required slidability (press resistance of the piston rod 5 being 12 N or less) cannot be obtained, and cracks may be generated in the COP syringe barrel depending on the width D of the slide-contact surface 11a. It should be noted that this range is basically not greatly influenced by the thickness of the syringe barrel 1.

In addition, PTFE not having the HIP treatment provided as described above has fine communicatively-connected gaps inside. By inserting the piston member 10 in the syringe barrel 1 and crushing the protruded rims 13 with the press-fit margin T, the fine communicatively-connected gaps are clogged during the cold flow described above, and not only leakage of liquid but also passage of vapor through the communicatively-connected gaps are stopped even with a PTFE not having the HIP treatment provided thereon.

When the relationship (press-fit margin T) between the internal diameter S of the syringe barrel 1 and the outer diameter of the liquid contact-side sliding part 16 of the piston member 10 is in the above range, and when the pitch P and the maximum height roughness Rz of the processed grooves 12 of the slide-contact surface 11a are in the above described ranges; the width D of the slide-contact surface 11a of the piston member 10 is set to 0.5 to 3 mm. When the width D is smaller than 0.5 mm, the liquid contact-side sliding part 16 may become damaged during handling because of being too narrow. When the width D is not smaller than 3 mm, the press-fit margin of the piston member 10 with respect to the syringe barrel 1 is larger than 50 µm, and when the syringe barrel 1 is made from COP; the surface pressure by the liquid contact-side sliding part 16 with respect to the syringe barrel 1 becomes too high, and the piston member 10 may expand larger than the syringe barrel 1 at normal temperature or during autoclaving at 121°C for 20 minutes to cause the syringe barrel 1 to crack. For practical use, 1 to 2 mm is suitable.

The points described above also apply to the middle piston 10b. In the middle piston 10b, as shown in FIG. 18, the liquid contact-side sliding parts 16 are formed on both ends, and the portion between the two liquid contact-side sliding parts 16 is formed to be narrow. The slide-contact surfaces 11a of the two liquid contact-side sliding parts 16 are formed in a completely identical manner as the slide-contact surface 11a of the gasket 10a.

The syringe barrel 1 has the needle mount part 1b at the front end, the finger placement part 1c at the back end, and a cylindrical drug solution loading part 4c formed therebetween; and is formed from cyclic polyolefin in the present example. It is needless to say that the shape of the syringe barrel 1 is not limited to those that are diagrammatically represented, and the material of the syringe barrel 1 may be polypropylene, glass, or the like.

The piston rod 5 is a rod shaped member having the male-screw part 5a formed at the front end part and a finger rest part 5b formed at the back end. On the outer circumferential surface of the male-screw part 5a of the piston rod 5, male-screw threads to be screwed in the female-screw hole 15 of the gasket 10a are engraved. The material of the piston rod 5 includes resins such as cyclic polyolefin, polycarbonate, and polypropylene.

The top cap 8 is attached to the needle mount part 1b of the syringe barrel 1, and is a sealing member to prevent leakage of the drug solution 30 loaded in the syringe barrel 1 and contamination of the drug solution 30 by unwanted germs drifting in air. The top cap 8 includes a cap main body 8a having a circular truncated cone shape, and an engagement protrusion 8c extending in an opening direction from a top surface of the cap main body 8a and having formed thereon a concaved portion 8b in which the needle mount part 1b is fitted. The top cap 8 is formed from an elastomer. Examples of the elastomer include both "thermoplastic elastomers" and "thermosetting elastomers" such as vulcanized rubbers and thermosetting resin based elastomers.

The pre-filled syringe A as shown in FIG. 1 is formed by assembling the members described above, and loading the drug solution 30 in the space enclosed between the syringe barrel 1 and the gasket 10a. When the pre-filled syringe A is used, usage preparation can be prepared only by taking off the top cap 8 and mounting a predetermined needle to the needle mount part 1b of the syringe barrel 1. During usage, the motion of the piston rod 5 is extremely smooth including the initial motion, and does not exceed 12 N as can be understood from the slide resistance test described later. Similarly, as can be understood from the liquid sealing-performance test, the gasket 10a can, with its excellent water repellency, prevent not only leakage of liquid but also passage of vapor between the slide-contact surface 11a and the inner circumferential surface 2 of the syringe barrel 1 over an extended period of time.

In the gasket 10a in FIG. 15, a cavity 18 is additionally formed inside a slide-contact surface 11 over the overall width of the slide-contact surface 11, by additionally performing an inner-boring process when forming the tap drill hole of the female-screw hole 15. With this, the thickness of the slide-contact portion is reduced to provide elasticity. As a result, touch by the syringe barrel 1 to the inner circumferential surface 2 becomes soft, and it becomes possible to press the piston rod 5 with lesser force. The male-screw part 5a of the piston rod 5 may or may not extend to make contact with the front end of the cavity 18. When a contact is made therebetween, the liquid contact surface 14 of the gasket 10a is reinforced.

In the gasket 10a in FIG. 20, the above described composite block 60 in which only the outer circumferential surface of the drug solution-resistant resin material 61 is covered with the PTFE member 62 is used. On the PTFE portion, the predetermined cut-processing described above is performed in accordance with the internal diameter S of the syringe barrel 1 to obtain the gasket 10a having the predetermined shape. The same applies to the middle piston 10b.

In the piston member 10 manufactured as described above, although the resin material 61 is exposed on the liquid contact surface 14, the drug solution 30 in contact thereto is not adversely effected since the resin material 61 has excellent drug solution-resistant property, such as COP and ultrahigh molecular weight polyethylene. Since the slide-contact surface 11a is formed from PTFE, excellent slidability is obtained without having leakages. It should be noted that when a resin harder than PTFE is selected as the drug solution-resistant resin material 61, PTFE at the outer circumference is reinforced from inside, and, since the drug solution-resistant resin material 61 ordinarily has a thermal expansion coefficient smaller than PTFE in a temperature range from around 0°C to near room temperature, the piston member 10 is not likely to be subjected to the influence of change in outside air temperature, and becomes superior than PTFE in terms of dimensional change associated with temperature.

FIG. 17 is another modification of the gasket 10a, and includes a main body portion 20, a liquid contact side slide-contact ring 23 fitted into a liquid contact-side outer circumferential surface 21 of the main body portion 20. Only the liquid contact side slide-contact ring 23 is formed from PTFE or a closed cell type PTFE. The liquid contact side slide-contact ring 23 is formed through cut-processing as described above. Then, the liquid contact side slide-contact ring 23 is fitted in a mold, and a drug solution-resistant resin that does not have an adverse effect with respect to the drug solution 30 is injected to form the gasket 10a having the liquid contact-side slide-contact cap 28 fitted on to the liquid contact-side sliding part 27 of the main body portion 26. It is needless to say that when the shape of the cavity of the mold is shaped as the middle piston 10b, the middle piston 10b having the liquid contact side slide-contact rings 23 fitted on the liquid contact-side outer circumferential surfaces 21 on both ends is obtained. Instead of injection molding, the liquid contact side slide-contact ring 23 may be fitted on the main body portion 20 that has been molded in advance. It is also possible to cut-process the main body portion 26, and then fit the liquid contact side slide-contact ring 23 on the liquid contact-side sliding part 27.

FIG. 18 is an example in which a bypass 1d is formed on the syringe barrel 1 in the example of the dual syringe B using the middle piston 10b. The middle piston 10b is housed in the syringe barrel 1, and the piston rod 5 having the gasket 10a mounted thereon is inserted on the flange 1c side of the syringe barrel 1. The middle piston 10b is disposed on the flange 1c side beyond an opening portion 1e of the bypass 1d located on the flange 1c side. The injection water 32 is loaded in the closed space between the middle piston 10b and the gasket 10a. The powdery drug 31 is loaded in the space formed between the needle mount part 1b and the middle piston 10b.

For usage, the top cap 8 is taken off and an injection needle is mounted on the needle mount part 1b, allowing immediate usage. When being used, the piston rod 5 is pressed, and the middle piston 10b advances via the loaded injection water 32. When the back end of the middle piston 10b passes the opening portion 1e of the bypass 1d on the flange 1c side, the injection water 32 passes through the bypass 1d and flows into the closed space filled with the drug 31 from an opening portion If on the front end side, and dissolves the drug 31 to be injected. In this case, the slide resistance is almost the same as that of the pre-filled syringe A in FIG. 1.

### Examples

In the following, the present invention will be described more specifically using Examples and Comparative Examples.

As shown in FIG. 1, pure water (placebo) that had been colored was loaded in a syringe barrel, and liquid sealing performance (leakage of liquid), vapor permeability test, and slidability (applied pressure to a piston rod) were tested with a gasket of the present invention made from PTFE using the following methods. The tested number in each of the tests was 10 each.

### Condition of Gasket of the Present Invention

Internal diameter (unit: mm) of syringe barrels (material: COP, glass)

| | | |
|---|---|---|
| = 6.34 | 12.45 | 20.20 |

Diameter (peak diameter H) of liquid contact-side sliding part (press-fit margin T: 40 µm) (unit: mm)

| | | |
|---|---|---|
| = 6.38 | 12.49 | 20.24 |

Pitch P of protruded rims (µm)

| | | | | | | | | |
|---|---|---|---|---|---|---|---|---|
| = 3 | 5 | 10 | 20 | 30 | 40 | 50 | 80 | 100 |

Maximum height roughness of processed groove: Rz (unit: µm)

| | | | |
|---|---|---|---|
| = 2.3 | 6.0 | 6.6 | 10 |

Width of slide-contact surface (unit: mm)

| | | | | |
|---|---|---|---|---|
| =0.5 | 1.0 | 1.5 | 2.5 | 3.5 |

### (1) Liquid Sealing Performance (Leakage of Liquid) Test

An interface portion between the slide-contact surface of the gasket and the inner circumferential surface of the syringe barrel was enlarged by 100 times to observe the presence of leakage of the placebo to the sliding portion. Those having a protruded rim with the pitch P of not larger than 50 µm and the maximum height roughness Rz of not larger than 6 µm did not generate leakage of liquid at room temperature even when the syringe barrel was made from PP, COP, or glass.

With all the internal diameters of the syringe barrel, leakage of liquid was observed when the pitches P of the protruded rims were 80 µm and 100 µm. Although a gasket whose protruded rims had the pitch P of 50 µm did not have any problems at room temperature, slight leakage of liquid was observed therein when a pre-filled syringe filled with placebo was kept overnight in a refrigerator at 5°C, and then returned to room temperature. Leakage of liquid was not observed with a gasket whose protruded rims had the pitch P of not larger than 40 µm. Although leakage of liquid was observed when the maximum height roughness Rz (groove depth) was 6.6 µm or 10 µm, leakage of liquid was not observed when the maximum height roughness Rz was not larger than 6 µm. The upper limits were a groove pitch of 50 µm and a maximum height roughness Rz (groove depth) of 6 µm. To be safe, the groove pitch is not larger than 40 µm and the maximum height roughness Rz (groove depth) is 3 µm.

### (2) Vapor Permeability Test (FIG. 23)

A syringe barrel made from glass (internal diameter: 6.34 mm, total volume: 2 ml) was filled with 1 ml of pure water that had been colored. On the syringe barrel, the PTFE gasket (the width D = 2.5 mm, diameter (peak diameter H) = 6.38 mm, press-fit margin =40 µm in slide-contact surface 11 a) manufactured in a shape that does not lead to leakage was mounted. Atop cap made from butyl rubber was mounted on the front end of the syringe barrel to measure weight change during the course of time. As a Comparative Example, a gasket made from butyl rubber commonly used for the syringe barrel was used.

A precision balance (manufactured by Shimadzu Corporation) used for measurement can measure a weight as small as 1/10,000 g.

Weight reduction of the syringe barrel having the gasket of the present invention used therein was almost 0 even after 1,500 hours, and the fluctuation range was within the error range of the precision balance. On the other hand, the conventional syringe barrel having used therein the gasket made from butyl rubber exhibited a weight reduction of 0.005 g.

### (3) Measurement of Slide Resistance Value (FIG. 24)

### Conditions of Gasket of the Present Invention

Diameter (peak diameter h) of liquid contact-side sliding part = 6.36 6.38 6.40 (unit: mm)
Width of slide-contact surface = 2.3 mm
Internal diameter of syringe barrel (COP, glass) = 6.34 mm
Testing rate = 100 mm/min.

The average slide resistances were 4.2 N with 6.36 mm, 5.9 N with 6.38 mm, and 7.4 N with 6.40 mm, and the average slide resistances were all 12 N or less as required. In the case where PTFE is used for the gasket 10a, when these average values were connected with a straight line and an intersection point with a horizontal line drawn from point where the slide-movement average (N) is 12 N was obtained, a press-fit margin of 150 µm is derived. Thus, it can be said that slidability is not compromised even when the press-fit margin in the diameter with respect to the syringe barrel 1 is increased to 150 µm. However, in the case with a COP syringe barrel, when the press-fit margin in the diameter is not smaller than 150 µm and the width D of the slide-contact surface 11 a is larger than 3 mm, cracks had sometimes occurred in the COP syringe barrel. Thus, the upper limit of the press-fit margin is 150 µm.

The lower limit is 10 µm when the relationships of cold flow of PTFE, groove depth, and pitch are considered. When the press-fit margin was not larger than 10 µm, the processed grooves could not be fully buried, and leakage had occurred.

### DESCRIPTION OF THE REFERENCE CHARACTERS

A: pre-filled syringe, B: dual syringe, D: width, P: pitch, Rz: maximum height roughness, Ra: arithmetical mean roughness, H: peak diameter, S: internal diameter of syringe barrel, T: press-fit margin, W: closed range adjacent to liquid contact surface, 1: syringe barrel, 1a: barrel main body, 1b: needle mount part, 1c: flange, 1d: bypass, 1e: opening on flange side, 1f: opening on front end side, 2: inner circumferential surface, 5: piston rod, 5a: male-screw part, 5b: finger rest part, 8: top cap, 8a: cap main body, 8b: concaved portion, 8c: engagement protrusion, 10: piston member, 10a: gasket, 10b: middle piston, 11: slide-contact surface, 11a: slide-contact surface on liquid contact surface side, 12: processed groove, 12a: groove bottom, 13: protruded rim, 13a: helical protruded rim, 13b: ring-shaped protruded rim, 14: liquid contact surface, 14a: liquid non-contact surface, 15: female-screw hole, 16: liquid contact-side sliding part, 17: end part of piston rod on mounting side, 18: cavity, 20: main body portion, 21: liquid contact-side outer circumferential surface, 23: liquid contact side slide-contact ring, 26: main body portion, 27: liquid contact-side sliding part, 28: liquid contact side slide-contact cap, 30: drug solution, 31: drug, 32: injection water, 50: PTFE bar material, 60: composite block, 61: drug solution-resistant resin material, 62: PTFE cylindrical material.

## Claims

1. A piston member formed by cut-processing a PTFE block and pressed and fitted in a syringe barrel to be used in a slidable manner,
the piston member comprising protruded rims formed at least in a circumferential direction of a whole circumference of a slide-contact surface that is adjacent to a liquid contact surface in contact with a drug solution, and that is a part of a slide-contact surface of the piston member, slidingly making contact with an inner circumferential surface of the syringe barrel, wherein
after the cut-processing, a pitch of the protruded rims is not larger than 50 µm, and a press-fit margin of the piston member with respect to the syringe barrel is 10 to 150 µm.

2. The piston member according to claim 1, wherein a maximum height roughness after the cut-processing is set to be not larger than 6 µm.

3. The piston member according to claim 1, wherein the protruded rims are helically formed.

4. The piston member according to claim 1, wherein the protruded rims are formed in a ring shape.

5. The piston member according to any one of claims 1 to 4, wherein a width of the slide-contact surface adjacent to the liquid contact surface of the piston member is 0.5 mm to 3 mm.

6. The piston member according to any one of claims 1 to 4, wherein the slide-contact surface adjacent to the liquid contact surface of the piston member is formed to be gradually smaller from a side of the liquid contact surface to a side of a liquid non-contact surface opposite thereto.

7. The piston member according to any one of claims 1 to 4, wherein the slide-contact surface adjacent to the liquid contact surface of the piston member is formed to be small, in a concaved arc shape, between a side of the liquid contact surface and a side of a liquid non-contact surface opposite thereto.

8. The piston member according to any one of claims 1 to 4, wherein the slide-contact surface adjacent to the liquid contact surface of the piston member is formed to be small, in concaved arc shapes, between a central portion of the liquid contact surface and a liquid non-contact surface opposite thereto, and both of the surfaces.

9. The piston member according to any one of claims 1 to 4, wherein a diameter of the piston member at the slide-contact surface adjacent to the liquid contact surface is set to be larger on a side of the liquid contact surface than a side of a liquid non-contact surface opposite thereto.

10. The piston member according to any one of claims 1 to 4, wherein at least three of the protruded rims counted from the liquid contact surface have the pitch, the maximum height roughness, and the press-fit margin set forth in claim 1, and the protruded rims subsequent to a fourth rim have at least one of the pitch, the maximum height roughness, and the press-fit margin larger than that of the three protruded rims on the side of the liquid contact surface.

11. The piston member according to any one of claims 1 to 4, wherein at least three of the protruded rims counted from the liquid contact surface have a ring shape, and the protruded rims subsequent to a fourth rim are helical.

12. The piston member according to any one of claims 1 to 4, wherein the whole piston member is formed from PTFE.

13. The piston member according to any one of claims 1 to 4, wherein the piston member is formed by cut-processing at least a PTFE portion of a composite block in which only an outer circumferential surface of a drug solution-resistant resin material is covered with a PTFE cylindrical material.

14. The piston member according to any one of claims 1 to 4, wherein a cavity is formed inside the slide-contact surface of the piston member.
